# Europäisches Patentamt
## European Patent Office
## Office européen des brevets

(19)

(11) Veröffentlichungsnummer: **0 070 797**
**A2**

(12) # EUROPÄISCHE PATENTANMELDUNG

(21) Anmeldenummer: **82810276.4**

(22) Anmeldetag: **28.06.82**

(51) Int. Cl.³: **B 01 J 10/00**
**// C07C85/12**

(30) Priorität: **16.07.81 CH 4659/81**

(43) Veröffentlichungstag der Anmeldung: **26.01.83**
**Patentblatt 83/4**

(84) Benannte Vertragsstaaten: **BE DE FR GB IT NL SE**

(71) Anmelder: **BUSS AG, Lautengartenstrasse 7,
CH-4052 Basel (CH)**

(72) Erfinder: **Süsstrunk, Hans-Ulrich, Käppelimattstrasse 6,
CH-4133 Pratteln (CH)**

(74) Vertreter: **Eder, Carl E. et al, Patentanwaltsbüro Eder &
Cie Münchensteinerstrasse 2, CH-4052 Basel (CH)**

(54) **Einrichtung mit einem Schleifenreaktor und Verfahren, bei dem ein flüssiges Edukt in einem Kreislauf einer chemischen Reaktion unterzogen wird.**

(57) Die Einrichtung weist einen mit einem Kreislauf (1) versehenen Schleifenreaktor für den chargenweisen Betrieb auf. Beim Chargen-Wechsel wird das für die nächste Charge benötigte, flüssige Edukt dem Kreislauf (1) über eine Zuleitung (43) zugeführt, in die in der Edukt-Strömungsrichtung gesehen, der Reihe nach ein erster Wärmeaustauscher (49), ein Entgasungs-Behälter (51), ein zweiter Wärmeaustauscher (55) und ein Zwischen-Behälter (57) eingeschaltet sind. Das im Kreislauf (1) gebildete Produkt wird über eine Ableitung (71) abgeleitet, in die der Reihe nach ein Zwischen-Behälter (75), der zweite Wärmeaustauscher (55) und der erste Wärmeaustauscher (71) eingeschaltet sind. Beim Chargen-Wechsel werden das Edukt und das Produkt vorübergehend in den Zwischen-Behältern (57, 75) gespeichert und im wesentlichen gleichzeitig durch die Wärmeaustauscher (49, 55) hindurchgeleitet, wobei das Produkt in den Wärmeaustauschern (49, 55) Wärme an das Edukt abgibt und das letztere auf eine zum Auslösen der Reaktion vorteilhafte Temperatur erhitzt. Dies ermöglicht, sowohl den Zeitbedarf für die Chargen-Wechsel als auch den Energieverbrauch niedrig zu halten.

0070797

Buss AG, Basel (Schweiz)

Einrichtung mit einem Schleifenreaktor und Verfahren, bei dem ein flüssiges Edukt in einem Kreislauf einer chemischen Reaktion unterzogen wird

_____

Die Erfindung betrifft eine Einrichtung gemäss dem Oberbegriff des Anspruchs 1.

Für die katalytische Hydrierung von flüssigen, organischen Edukten mit Wasserstoff und auch für andere chemische Reaktionen, bei denen ein flüssiges Edukt und ein gasförmiges Edukt zur Reaktion gebracht werden, verwendet man, insbesondere wenn grössere Produktemengen herzustellen sind, häufig Schleifenreaktoren. Derartige Schleifenreaktoren und Anwendungen davon sind unter anderem aus den schweizerischen Patentschriften 370 057 und 405 240, der österreichischen Patentschrift 287 890 sowie der Publikation "Loop Reactor Technology Improves Catalytic Hydrogenations" von R.J.Malone, Chemical Engineering Progress, Juni 1980, Seiten 53 bis 59 bekannt.

Ein Schleifenreaktor der zurzeit gebräuchlichen Art weist einen Reaktions-Behälter auf, in den oben eine Injektor-Mischdüse hineinragt. Im Boden des Reaktions-Behälters ist ein Auslass vorhanden, der über eine Leitung, in die eine Umwälzpumpe und ein Wärmeaustauscher eingeschaltet ist, mit der Mischdüse verbunden ist. Der Behälter bildet also zusammen mit der erwähnten Leitung und den in diese eingeschalteten Organen einen Kreislauf. Durch in den Kreislauf mündende Zuleitungen kann diesem ein flüssiges Edukt, ein gasförmiges Edukt und ein Katalysator zugeführt werden. Das bei der Reaktion gebildete Produkt kann durch eine vom Kreislauf abgezweigte Ableitung entnommen werden.

16139/Zb/sr/Fall 806

Nun soll anhand eines Beispiels, nämlich der Hydrierung eines Nitrils, die Arbeitsweise eines bekannten Schleifenreaktors erläutert werden. Zunächst wird der Behälter gespült und danach eine Charge, d.h. eine bestimmte vorgegebene Menge des Nitrils in den Behälter eingebracht und im Kreislauf zirkuliert. Dem in den Kreislauf eingeschalteten Wärmeaustauscher wird dabei durch einen sekundären Kreislauf Wärme zugeführt, wobei im Sekundär-Kreislauf ein Wärmeträger, zum Beispiel Wasserdampf bzw. flüssiges Wasser zirkuliert wird. Das Nitril wird beim Passieren des Wärmeaustauschers aufgewärmt und nötigenfalls getrocknet und mittels einer mit dem Behälter verbundenen Saugvorrichtung entgast. Ferner wird nun gasförmiger Wasserstoff zugeführt, so dass im oberen Teil des Behälters eine Wasserstoffatmosphäre vorhanden ist, und auch der Katalysator in den Kreislauf eingebracht. Wenn die zirkulierende Flüssigkeit auf eine gewisse, im Bereich von 150 bis 170°C liegende Temperatur aufgeheizt ist, setzt die chemische Reaktion ein. Die chemische Reaktion läuft dabei vor allem im Bereich der Mischdüse ab, die ähnlich wie eine Wasserstrahlpumpe ausgebildet ist und Wasserstoff ansaugt sowie in engen Kontakt mit der Flüssigkeit bringt.

Da die Reaktion exotherm ist, wird es bald nach dem Einsetzen der Reaktion notwendig, den Kreislauf zu kühlen. Zu diesem Zweck wird der in den Kreislauf eingeschaltete Wärmeaustauscher auf Kühlbetrieb umgeschaltet, wozu dem Wärmeaustauscher durch den Sekundär-Kreislauf kaltes Wasser zugeführt wird.

Wenn die ganze Nitril-Charge hydriert ist, wird das entstandene, flüssige Produkt, im vorliegenden Fall ein Amin, noch während einer gewissen Zeit im Kreislauf zirkuliert und abgekühlt. Wenn die Temperatur auf eine die Filtration ermöglichenden Wert abgesunken ist, wird das Produkt durch die Ableitung aus dem Kreislauf in einen Filtrations-Zwischenbehälter abgeleitet und danach mit einer Filterpresse filtriert und vom Katalysator getrennt.

In der vorgängig zitierten Publikation von R.J. Malone ist als Anwendungsbeispiel eines Schleifenreaktors die Bildung von 2-Chloranisidin durch Hydrieren erwähnt. Bei einem für eine Jahresproduktion von 1800 Tonnen konzipierten Schleifenreaktor dauert ein für die Verarbeitung einer Charge erforderlicher Zyklus insgesamt 164 Minuten. Dabei erfordert das Evakuieren und Spülen des Behälters 15 Minuten, das Einfüllen des flüssigen Edukts 10 Minuten, die Zugabe des Katalysators 5 Minuten, das Aufheizen 20 Minuten, die Durchführung der Reaktion 74 Minuten, das Abkühlen 30 Minuten und das Ableiten des gebildeten Produktes 10 Minuten. Beim Betrieb des aus der genannten Publikation bekannten Schleifenreaktors beträgt also die für die Verarbeitung einer Charge insgesamt benötigte Zeit mehr als das Doppelte der eigentlichen Reaktionszeit. Ein so grosses Verhältnis der gesamten Verarbeitungszeit zur eigentlichen Reaktionszeit ist natürlich unerwünscht und macht bei einer vorgegebenen Jahresproduktionsrate die Verwendung eines relativ grossen Behälters notwendig. Bei der Herstellung vieler anderer Produkte aus einem flüssigen und einem gasförmigen Edukt ergeben sich ähnliche Verhältnisse.

Ein anderer Nachteil des vorbekannten Schleifenreaktors besteht auch noch darin, dass zum Aufheizen des flüssigen Eduktes auf die zum Auslösen der Reaktion erforderliche Temperatur eine beträchtliche Energiemenge

0070797

erfoderlich ist. Diese wird von einer äusseren Energiequelle aufgebracht und kann grosse Kosten verursachen.

Da viele der in Schleifenreaktoren durchgeführten Reaktionen, wie die erwähnten Hydrierungen, exotherm verlaufen, muss die im Kreislauf zirkulierende Flüssigkeit, wie bereits dargelegt, im Wärmeaustauscher bis
zum Einsetzen der Reaktion erwärmt und danach gekühlt
werden. Bei gewissen Reaktionen setzt die Wärmeabgabe
ziemlich schlagartig und heftig ein, so dass der
Wärmeaustauscher entsprechend schnell vom Heizbetrieb
auf den Kühlbetrieb umgeschaltet werden sollte. Diese
Umschaltung vom Heiz- auf den Kühlbetrieb wird jedoch
dadurch verzögert, dass der Wärmeaustauscher und Teile
des damit verbundenen SekundärKKreislaufes eine gewisse Wärmekapazität aufweisen und zuerst selbst abgekühlt werden müssen. Wenn also der in den Kreislauf
eingeschaltete Wärmeaustauscher zuerst Wärme zuführen
und danach Wärme abführen muss, bringt dies die Gefahr
mit sich, dass die Temperatur beim Einsetzen der Reaktion unerwünscht hoch ansteigt. Dieses wiederum kann zur
Folge haben, dass die Reaktionsgeschwindigkeit zu gross
wird und die Kontrolle über den Reaktionsablauf verloren
geht, so dass der Reaktionsvorgang gewissermassen "durchgeht", wobei die Gefahr besteht, dass unerwünschte
Nebenprodukte entstehen, die sehr toxisch sein und/oder
zur Explosion neigen können.

Aus der Publikation "Funktionseinheiten zur katalytischen Hydrierung und Reduktion" vom Mai 1972 der
Buss AG, Basel ist eine Hydrierungs-Einrichtung bekannt,
die eine aus mindestens zwei hintereinander geschalteten
Schleifenreaktoren gebildete Kaskade aufweist. Jeder
Schleifenreaktor weist einen Behälter und eine mit diesem
zusammen einen Kreislauf bildende Leitung auf, die vom Boden des Behälters über eine Umwälzpumpe und einen Wärmeaustauscher zu einer wieder in den Behälter mündenden Mischdüse führt. Das flüssige Edukt wird dem ersten Schleifenreaktor über einen Wärmeaustauscher zugeführt. In der Anlaufphase wird die Reaktionsflüssigkeit im Kreislauf durch die ganze Kaskade zirkuliert und in den zu den einzelnen Schleifenreaktoren gehörenden Wärmeaustauschern erhitzt, bis die Reaktionstemperatur erreicht ist. Danach kann die Einrichtung kontinuierlich arbeiten, wobei die Reaktionsflüssigkeit der Reihe nach die zwei oder mehr Schleifenreaktoren durchläuft. Das gebildete Produkt wird vom zweiten bzw. letzten Schleifenreaktor über eine Ableitung und einen Durchgang des Wärmeaustauschers, über den das Edukt dem ersten Schleifenreaktor zugeführt wird, einem Entgasungsbehälter zugeführt. Von diesem wird es dann zu einem Filter weitergeleitet.

Eine derartige, mit mindestens zwei Schleifenreaktoren ausgerüstete Einrichtung kann an sich nach der erwähnten Anlaufphase kontinuierlich arbeiten. Bei der praktischen Anwendung von Schleifenreaktoren werden diese jedoch meistens abwechselnd für die Herstellung verschiedener Produkte verwendet und sind im allgemeinen höchstens einige wenige Tage ununterbrochen in Betrieb. Dementsprechend ergeben sich häufig Betriebsunterbrüche, wobei dann bei jeder Wiederaufnahme des Betriebes zuerst eine Anlaufphase notwendig wird.

Bei einem Produktewechsel müssen die mit dem Produkt in Berührung gelangenden Teile sauber gereinigt werden. Bei Einrichtungen mit zwei oder mehr Schleifenreaktoren muss natürlich jeder Schleifenreaktor gereinigt werden, so dass der Aufwand für die Reinigung verhältnismässig gross wird.

Des weitern ist bei Einrichtungen mit einer Kaskade von Schleifenreaktoren auch der apparative Aufwand grösser als bei einer Einrichtung mit einem einzelnen, diskontinuierlich betriebenen Schleifenreaktor. Hier ist insbesondere darauf hinzuweisen, dass die in die Kreisläufe der Reaktions-Schleifen eingeschalteten Pumpen sehr leistungsfähig sein und starken Beanspruchungen widerstehen müssen.

Eine für den kontinuierlichen Betrieb vorgesehene Einrichtung mit zwei oder mehr Schleifenreaktoren ist daher sowohl was die Herstellungskosten als auch was die Betriebskosten anbelangt, im allgemeinen teurer als eine für den Chargen-Betrieb vorgesehene, nur einen Schleifen-Reaktor aufweisende Einrichtung mit gleicher mittlerer Produktionsleistung. In der Praxis werden daher nur verhältnismässig selten Einrichtungen mit Kaskaden von Schleifenreaktoren eingesetzt.

In der US-Patentschrift 2 232 674 ist eine Einrichtung für die Durchführung chemischer Reaktionen von flüssigen Edukten, insbesondere für die Alkylierung von Isoparaffinen mit Olefinen, geoffenbart. Die Einrichtung weist eine einen Kreislauf bildende Leitung auf, in die ein Tank mit perforierten Platten eingeschaltet ist. Ein Teil der im Kreislauf zirkulierenden Flüssigkeit wird durch eine Temperatur-Regeleinheit hindurch geführt, die je nach Bedarf als Heizer oder Kühler ausgebildet sein kann. Die verschiedenen Edukte werden über eine gemeinsame Zuleitung, einen Wärmeaustauscher, eine zur Abscheidung unerwünschter Komponenten, wie Wasser, dienenden Vorrichtung und einen Mischer dem Kreislauf zugeführt. Das im Kreislauf gebildete Produkt wird über eine Ableitung, einen Separator, den bereits erwähnten Wärmeaustauscher und einen weitern Separator abgeleitet.

Gemäss der US-Patentschrift 2 232 674 wird diese bekannte Einrichtung offensichtlich kontinuierlich betrieben. Die Einrichtung wäre jedoch auch gar nicht für einen chargenweisen Betrieb geeignet, weil es nämlich nicht möglich wäre, im Wärmeaustauscher einen Wärmeaustausch zwischen dem abgeleiteten Produkt und den zugeführten Edukten vorzunehmen, wenn das Produkt aus dem Kreislauf abgeleitet würde, bevor die Edukte zugeführt werden.

Die kontinuierliche Arbeitsweise der Einrichtung gemäss der US-Patentschrift 2 232 674 bringt ferner den Nachteil mit sich, dass das aus dem Kreislauf abgeleitete Produkt noch einen Anteil Edukte enthält, die in den erwähnten Separatoren abgetrennt und wieder dem Kreislauf zugeführt werden müssen. Im übrigen ist die Einrichtung weder ausgerüstet noch geeignet, um ein gasförmiges Edukt in den Kreislauf einzuführen und in diesem mit einem flüssigen Edukt zur Reaktion zu bringen.

Aus der englischen Patentschrift 1 130 330 ist ein Reaktor für die Phenol-Formaldehyd-Harz-Herstellung bekannt. Die Einrichtung weist einen Tank für die Speicherung einer Phenol-Formaldehyd-Mischung, einen Vorheiz-Kreislauf, einen Reaktor-Kreislauf, einen Kühl-Kreislauf und ein Auslass-System auf. Der Tank, die drei Kreisläufe und das Auslass-System sind in dieser Reihenfolge miteinander verbunden und in jeden Kreislauf ist ein Wärmeaustauscher und ein Ventil eingeschaltet. Ferner ist ein Temperatur-Regelsystem vorhanden, das mit Durchgängen der Wärmeaustauscher des Vorheiz- und des Reaktor-Kreislaufs verbunden ist. Beim Betrieb wird das Phenol-Formaldehyd-Gemisch in den Kreisläufen zirkuliert und chargenweise weitergeleitet.

Bei dieser bekannten Einrichtung ist kein sowohl von einem zugeführten Edukt als auch von einem abgeleiteten Produkt durchströmter Wärmeaustauscher vorhanden. Insbesondere wird das Produkt nach der Reaktion offensichtlich im erwähnten Kühlkreislauf gekühlt, von dem Wärme nach aussen abgeführt und nicht auf das Edukt übertragen wird. Es wäre also allerhöchstens möglich, dass mit dem erwähnten Temperatur-Regelsystem Wärme von dem in den Reaktor-Kreislauf eingeschalteten Wärmeaustauscher zu dem in den Vorheiz-Kreislauf eingeschalteten Wärmeübertrager und damit von den im Reaktor-Kreislauf zirkulierenden Substanzen auf das Edukt-Gemisch übertragen wird.

Die aus der englischen Patentschrift 1 130 330 bekannte Einrichtung ist zudem sehr kompliziert und hat ähnliche Nachteile wie die vorgängig schon beschriebene, aus der Publikation der Buss AG bekannte, kontinuierlich arbeitende, eine Schleifenreaktor-Kaskade aufweisende Einrichtung. Im übrigen ist die Einrichtung gemäss der englischen Patentschrift 1 130 330 auch nicht ausgerüstet, um dem Reaktions-Kreislauf ein gasförmiges Edukt zuzuführen und dieses mit einem flüssigen Edukt zur Reaktion zu bringen.

Es sei auch noch auf die Schweizer-Patentschrift 359 819 verwiesen. Aus dieser ist eine Einrichtung für die selektive Härtung von ungesättigten Ölen und Fetten bekannt. Die Einrichtung weist einen Autoklav auf, der durch Zwischenwände in eine Reihe nebeneinander angeordneter Kammern unterteilt ist. Die Zwischenwände sind mit Überlauf-Öffnungen versehen, so dass das Öl beim Erreichen eines vorgegebenen Niveaus von einer Kammer in die nächste überströmen kann. Das zu härtende Öl wird der ersten Kammer über eine Öl-Zuleitung zugeführt, in die in der Strömungsrichtung gesehen der Reihe nach ein Dampferhitzer, ein Wärmeaustauscher und nochmals ein Dampferhitzer eingeschaltet sind. Zwischen dem Trockner und dem zweitgenannten Dampferhitzer mündet eine Katalysator-Speiseleitung in die Öl-Zuleitung.

Das der ersten Autoklav-Kammer zugeführte Öl strömt sukzessive durch die ganze Reihe der Autoklav-Kammern, wobei es in jeder Kammer in einem Kreislauf zirkuliert, der eine Umwälzpumpe, einen Kühler und einen Mischer aufweist. Von der letzten Kammer wird das Öl über eine Ableitung durch den bereits erwähnten Wärmeaustauscher einem mit einem Rührer ausgerüsteten Behälter zugeführt. Vom letzteren wird das Öl dann zu Filterpressen weitergeleitet. Im Wärmeaustauscher findet eine Wärmeübertragung von dem aus der letzten Kammer abgeleitete Öl auf das in die erste Kammer eingeleitete Öl statt.

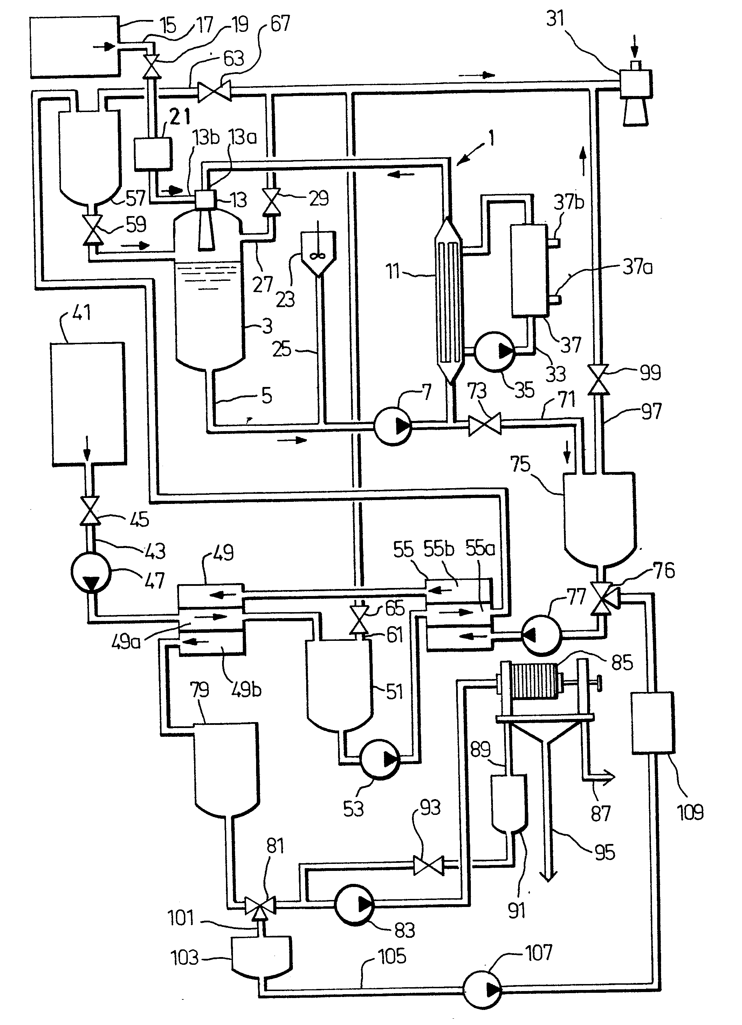

Die aus der Schweizerpatentschrift 359 819 bekannte Einrichtung arbeitet also kontinuierlich. Da der Autoklav jedoch in eine relativ grosse Anzahl Kammern unterteilt ist, nämlich gemäss der Zeichnung in sechs Kammern, und da für jede dieser Kammern ein Kreislauf vorhanden sein muss, ist der apparative Aufwand für eine derartige Einrichtung sehr gross. Des weitern ist die Reinigung dieser Einrichtung, und insbesondere des in Kammern unterteilten Autoklavs, sehr schwierig und zeitraubend. Obschon ferner ein Teil der durch die chemischen Reaktionen erzeugten Wärme im erwähnten Wärmeaustauscher vom abgeleiteten Öl auf das zugeleitete Öl übertragen werden kann, muss das zugeleitete Öl zusätzlich noch in zwei Dampferhitzern erwärmt werden. Die Durchführung des Verfahrens erfordert also auch sehr viel von äusseren Energiequellen zugeführte Energie.

Der Erfindung liegt nun ausgehend von der Einrichtung

0070797

gemäss der US-Patentschrift 2 232 674 die Aufgabe zugrunde, eine Einrichtung mit einem Schleifenreaktor und einem Wärmeaustauscher mit in die Edukt-Zuleitung und die Produkt-Ableitung eingeschalteten Durchgängen zu schaffen, wobei die Einrichtung einen chargenweisen Betrieb ermöglichen soll.

Diese Aufgabe wird durch eine Einrichtung der einleitend genannten Art gelöst, wobei die Einrichtung gemäss der Erfindung durch die Merkmale des Anspruchs 1 gekennzeichnet ist.

Zweckmässige Ausgestaltungen der Einrichtung ergeben sich aus den Ansprüchen 2 bis 6.

Die Erfindung betrifft ferner ein Verfahren gemäss dem Oberbegriff des Anspruchs 7. Das Verfahren ist erfindungsgemäss durch die Merkmale des Anspruchs 7 gekennzeichnet.

Besonders zweckmässige Ausgestaltungen des Verfahrens gehen aus den Ansprüchen 8 bis 10 hervor.

Der Erfindungsgegenstand soll nun anhand eines in der Zeichnung dargestellten Ausführungsbeispiels erläutert werden. Die einzige Figur der Zeichnung zeigt ein Schema einer Einrichtung mit einem Schleifenreaktor.

In der einzigen Figur der Zeichnung ist eine Einrichtung mit einem einstufigen Schleifenreaktor, d.h. nur einem einzigen Schleifenreaktor ersichtlich, dessen Schleife bzw. Primär-Kreislauf 1 durch einen Reaktions-Behälter 3, nämlich einen Autoklav und eine Schleifen-Leitung 5 gebildet wird. Die letztere verbindet eine im Boden des Behälters 3 vorhandene Öffnung über eine Flüssigkeits-Pumpe 7 und ein Wärmeübertragungsorgan 11, nämlich einen Primär-Kreislauf-Wärmeaustauscher, mit dem einen Eingang-Anschluss 13a einer Injektor-Mischdüse 13, die bei der Deckwand des Behälters 3 von oben her in diesen einmündet.

Eine Speisequelle 15 für ein gasförmiges Edukt, insbesondere Wasserstoff, ist durch eine Zuleitung 17, in die ein Sperr- und Regelventil 19 sowie Gaszähler 21 eingeschaltet ist, mit einem Eingangsanschluss 13b der Injektor-Mischdüse 13 verbunden. Die letztere ist ähnlich wie eine Wasserstrahlpumpe ausgebildet, so dass sie beim Betrieb der Einrichtung während der Reaktionsphase aus der Leitung 17 und der im obersten Teil des Behälters 3 vorhandenen Gas-Atmosphäre Gas ansaugt und in engen Kontakt mit der die Düse durchströmenden Flüssigkeit bringt.

Ein Katalysator-Speiseorgan 23 ist über eine Leitung 25 mit dem Kreislauf 1 verbunden. Dabei mündet die Leitung 25 in den den Behälter 3 mit der Saugseite der Pumpe 7 verbindenden Abschnitt der Leitung 5. Das Sperr- und Dosiermittel, wie Ventile, aufweisende Katalysator-Speiseorgan 23 ermöglicht, der beim Betrieb im Kreislauf 1 zirkulierenden Flüssigkeit einen suspendierten Feststoff-Katalysator beizufügen.

Die Pumpe 7 ist in bekannter Weise mit zwei Dichtungen ausgerüstet, von denen die eine einen zwischen den beiden Dichtungen vorhandenen Zwischenraum gegen die durch den Kreislauf 1 gepumpte Flüssigkeit und die andere den genannten Zwischenraum gegen die Umgebung abdichtet. Der zwischen den beiden Dichtungen vorhandene Zwischenraum ist in ebenfalls bekannter Weise in einen nicht dargestellten Kreislauf einer als Sperr- und Kühlmittel dienenden Flüssigkeit eingeschaltet.

Der oberste Teil des Innenraumes des Behälters 3 ist über eine ein Sperrventil 29 enthaltende Leitung 27 mit einer Vakuum-Saugpumpe 31 verbunden, die beispielsweise, wie in der Zeichnung angedeutet, als Strahlpumpe ausgebildet sein kann.

Das in den Primär-Kreislauf eingeschaltete Wärmeübertragungsorgan 11 ist, wie bereits erwähnt, durch einen Wärmeaustauscher gebildet und weist abgesehen von seinen in den Kreislauf 1 eingeschalteten Durchgang noch einen Durchgang auf, dessen Enden mit einer einen Sekundär-Kreislauf 33 bildende Leitung verbunden sind. In diesen nur vereinfacht dargestellten Sekundär-Kreislauf 33 ist eine Pumpe 35 und ein Wärmeregelorgan 37 eingeschaltet. Das letztere weist mindestens einen Kühler auf. Eventuell ist das Wärmeregelorgan 37 zusätzlich zum Kühler noch mit einem Erhitzer und mit Ventilen ausgestattet, um wahlweise den Kühler oder den Erhitzer in den Sekundär-Kreislauf einzuschalten, in dem bei der Benutzung Druckwasser zirkuliert wird. Der Kühler, der seinerseits wieder als Wärmeaustauscher ausgebildet ist, kann mit Anschlüssen 37a, 37b verbunden sein oder über Ventile mit diesen verbunden werden. Der Kühler kann über diese Anschlüsse 37a, 37b in einen externen Wärmemittel-Kreislauf eingeschaltet werden, so dass die während der Reaktionsphase anfallende und über das Wärmeübertragungsorgan 11 abgegebene Energie einem externen Wärmeverbraucher zugeführt werden kann.

Es sei jedoch bemerkt, dass anstelle des beschriebenen Sekundär-Kreislaufes ein Kondensatsammelbehälter vorhanden sein könnte, der derart ausgebildet und mit dem Wärmeübertragungsorgan 11 verbunden ist, dass er dem letzteren zum Kühlen eine Flüssigkeit zuführt, die dann verdampft und nach erfolgter Kondensation im Sammelbehälter wieder gesammelt wird.

Das für die Durchführung der vorgesehenen chemischen Reaktion benötigte, flüssige, organische Edukt kann von einer schematisch als Behälter dargestellten Speisequelle 41 über eine Zuleitung 43 in den Behälter 3 eingeführt werden, und zwar in dessen oberen Innenraum-Teil. In die Zuleitung 43 ist, beginnend bei der Speisequelle 41, ein Ventil 45, eine Pumpe 47, ein Durchgang 49a eines ersten Wärmeaustauschers 49, ein Entgasungs-Behälter 51, eine Pumpe 53, ein Durchgang 55a eines zweiten Wärmeaustauschers 55, ein wärmeisolierter Zwischen-Behälter 57 und ein Ventil 59 eingeschaltet. Der Zwischen-Behälter 57 ist dabei oberhalb der Einmündung der Zuleitung 43 in den Reaktions-Behälter 3 angeordnet und die genannte Einmündung befindet sich oberhalb des beim Betrieb im Behälter 3 vorhandenen Flüssigkeitsniveaus, so dass das zugeführte Edukt unter der Einwirkung der Schwerkraft vom Zwischenbehälter 57 in den Reaktions-Behälter 3 strömen kann.

Die obersten Innenraum-Bereiche der Behälter 51 und 57 sind über Leitungen 61 bzw 63, in die ein Sperrventil 65 bzw. 67 eingeschaltet ist, mit der Saugpumpe 31 verbunden.

Vom Kreislauf 1, nämlich von dem den Ausgang der Pumpe 7 mit dem Wärmeübertragungsorgan 11 und dem Behälter 3 verbindenden Abschnitt der Leitung 5, zweigt eine zum Ableiten des gebildeten Produks aus dem Kreislauf 1 dienende Ableitung 71 ab. In diese sind, beginnend von der Abzweigung aus dem Kreislauf 1 her, ein Sperrventil 73, ein vorzugsweise wärmeisolierter, sowie zweckmässigerweise unterhalb des Kreislaufs 1 angeordneter Zwischen-Behälter 75, ein Dreiwegventil 76, eine Pumpe 77, ein Durchgang 55b des bereits erwähnten Wärmeaustauschers 55, ein Durchgang 49b des ebenfalls bereits erwähnten Wärmeaustauschers 49, ein Zwischen-Behälter 79, ein Dreiwegventil 81, eine Filterpress-Pumpe 83 und eine Filterpresse 85 eingeschaltet. Durch die an einen Anschluss der letzteren angeschlossene Leitung 87 kann beim Betrieb das filtrierte, reine Produkt entnommen werden. Ein anderer Anschluss der Filterpresse 85 ist über eine Leitung 89, einen Vorlauf-Behälter 91 und ein Sperrventil 93 mit demjenigen Abschnitt der Ableitung 71 verbunden, der das Dreiwegventil 81 mit der Pumpe 83 verbindet. Ein anderer Anschluss der Filterpresse 85 ist mit einer Leitung 95 verbunden, aus der der aus dem Produkt abgeschiedene Katalysator entnommen werden kann. Der abgeschiedene Katalysator kann, eventuell nach einer zusätzlichen Aufbereitung, für die Wiederverwendung wieder in das Katalysator-Speiseorgan 23 gebracht werden. Der Zwischen-Behälter 75 ist über eine Leitung 97 mit einem Sperrventil 99 mit der Saugpumpe 31 verbunden.

Ein Anschluss des Dreiwegventils 81 ist durch eine Zweigleitung 101 mit einem Spühlmittel-Reservoir 103 verbunden. Dieses weist im Bereich seines Bodens einen Auslass auf, der über eine Leitung 105, in die eine Pumpe 107 und ein Heizorgan 109 eingeschaltet sind, mit dem Dreiwegventil 76 verbunden ist. Der Einlass der Pumpe 77 kann über das Ventil 76 wahlweise mit dem Behälter 75 oder dem Heizorgan 109 verbunden werden. Dabei sind das Ventil 76 und/oder die Pumpen 77 und 107 derart ausgebildet, dass der Zufluss zum Wärmeaustauscher-Durchgang 55b vollständig gesperrt werden kann. Im übrigen sind die beiden Zwischen-Behälter 57 und 75 derart bemessen, dass der Behälter 57 eine ganze Edukt-Charge und der Behälter 75 eine aus einer ganzen Edukt-Charge gebildete Produktmenge aufnehmen können.

Im folgenden soll die Arbeitsweise der Einrichtung erläutert werden. Dabei wird als Beispiel der Fall betrachtet, dass als gasförmiges Edukt Wasserstoff und als flüssiges Edukt aliphatisches Nitril zugeführt werden. Als Katalysator wird Nickel aufgeschlämmt. Das Nitril wird im Schleifenreaktor hydriert, so dass ein Amin entsteht.

Nun wird angenommen, der Reaktions-Behälter 3 sei wegen eines Betriebsunterbruches oder wegen eines Wechsels des herzustellenden Produktes vorgängig entleert worden, so dass also der Schleifenreaktor neu in Betrieb gesetzt werden muss. Dazu wird der Kreislauf 1 zunächst mit der Saugpumpe 31 evakuiert. Danach wird eine erste Charge Nitril aus der Speisequelle 41 durch die Zuleitung 43 und die in diese eingeschalteten Organe in den Reaktions-Behälter 3 eingeleitet, so dass dieser zu beispielsweise ungefähr 70 % gefüllt wird. Dabei ergibt sich im Behälter ein Flüssigkeits-Niveau, das unterhalb der Einmündung der Zuleitung 43 liegt. Das von der Speisequelle 41 gelieferte Nitril kann beispielsweise Raumtemperatur oder eventuell eine bestimmte Lagerungstemperatur aufweisen. Das Nitril strömt beim Zuführen unter anderem durch die Durchgänge 49a und 55a der beiden Wärmeaustauscher 49 bzw. 55.

Ferner wird gleichzeitig aus dem Reservoir 103 flüssiges Spülmittel durch das Heizorgan 109 hindurch gefördert. Das im Heizorgan 109 erhitzte Spülmittel wird dann durch die Pumpe 77 durch die Durchgänge 55b und 49b der Wärmeaustauscher 55 bzw. 49 hindurch gepumpt und gelangt danach über den Behälter 79 und das Ventil 81 in das Reservoir 103 zurück. Das die Wärmeaustauscher 55 und 49 im Gegenstrom zum Nitril durchströmende Spülmittel gibt Wärme an das Nitril ab. Die Durchflussmenge und Temperatur des durch die Pumpe 77 zugeführten Spülmittels sind dabei derart auf die Durchflussmenge und Anfangstemperatur des Nitrils abgestimmt, dass dieses mit einer Temperatur von ungefähr $110^{\circ}$ C in den Entgasungs-Behälter gelangt, wo die leichtflüchtigen Beimengungen entweichen können und mittels der Saugpumpe 31 abgesaugt werden. Danach wird das Nitril durch den Durchgang 55a des Wärmeaustauschers 55 gepumpt, wobei es auf eine Temperatur von beispielsweise ungefähr $175^{\circ}$ C erhitzt wird.

Sobald die erste Nitril-Charge vollständig in den Reaktions-Behälter 3 eingebracht ist, wird das Ventil 59 geschlossen und eine zweite Nitril-Charge mit dem erhitzten Spülmittel in den Wärmeaustauschern 49, 55 vorgewärmt, dem Zwischenbehälter 57 zugeführt und in diesem gespeichert. Gleichzeitig kann das Nitril der ersten Charge mittels der Pumpe 7 in der Richtung der Pfeile im Kreislauf 1 zirkuliert werden, wobei dem Nitril aus dem Katalysator-Speiseorgan 23 dosiert Katalysator zugeführt wird. Nachdem nach der Evakuierung des Behälters 3 das Ventil 29 wieder geschlossen wurde, wird aus der Speisequelle 15 ferner durch die Mischdüse 13 hindurch Wasserstoffgas in den Behälter 3 eingeleitet, so dass im letzteren oberhalb des Flüssigkeitsniveaus eine Wasserstoff-Atmosphäre entsteht.

Da das Nitril in den Wärmeaustauschern 49, 55 auf eine zum Auslösen der Hydrierung ausreichend hohe Temperatur erhitzt wurde, kann der Hydrierungsvorgang sogleich beginnen, wenn das Nitril und der diesem in Form einer Suspension beigefügten Katalysator im Kreislauf 1 zirkuliert werden. Der bei der Zirkulation in der Injektor-Mischdüse 13 entstehende Flüssigkeitstrahl saugt dabei Wasserstoff aus der im Behälter 3 vorhandenen Wasserstoff-Atmosphäre an und bringt diesen Wasserstoff in engen Kontakt mit dem Nitril, so dass die chemische Reaktion hauptsächlich in der Mischdüse 13 stattfindet. Der bei der Hydrierung verbrauchte Wasserstoff wird dabei über das Ventil 19 und den Gaszähler 21 nachgeliefert, wobei die Zufuhr derart geregelt wird, dass im Behälter 1 der vorgesehene Betriebsdruck aufrechterhalten bleibt.

Wenn der chemische Reaktionsvorgang gestartet wird, braucht der zirkulierten Flüssigkeit im Wärmeübertragungsorgan 11 zunächst weder Wärme zugeführt noch Wärme entnommen zu werden. Da die Hydrierung jedoch stark exotherm abläuft, erhöht sich nach dem Start des Reaktionvorganges die Temperatur der zirkulierten Flüssigkeit. Die durch die Reaktion erzeugte Wärme wird nun in dem als Kühler arbeitenden Wärmeübertragungsorgan 11 durch im Sekundärkreislauf 33 zirkulierendes Druckwasser abgeführt. Die Wärmeabfuhr wird dabei derart geregelt, dass die Temperatur auf beispielsweise $\mp 1^{\circ}$ genau auf einen vorgegebenen Wert konstant gehalten wird, der in einem um $200^{\circ}$ liegenden Grössenbereich liegt. Die abgeführte Reaktionswärme wird im Kühler des Wärmeregelorgans 37 beispielsweise an Kühlwasser abgegeben, oder durch Verdampfen von Kondensat abgeführt. Die Wärme kann im Bedarfsfall mittels des Dampfes an externe Wärmeverbraucher abgegeben werden, die an die Anschlüsse 37a, 37b angeschlossen sind und zusammen mit dem zum Wärmeregelorgan 37 gehörenden Kühler einen weitern Kreislauf bilden.

Sobald der Reaktionsvorgang abgeschlossen ist, d.h. wenn die ganze zuerst zugeführte Charge des flüssigen Eduktes in das gewünschte Produkt, nämlich in Amin umgewandelt wurde, werden die Wasserstoff- und Katalysatorzufuhr gestoppt und das gebildete Roh-Produkt aus dem Kreislauf 1 entnommen sowie eine neue Edukt-Charge in den Kreislauf eingebracht. Zur Entnahme des flüssigen, noch Katalysator-Beimengungen enthaltenden Roh-Produktes wird das Ventil 73 geöffnet, so dass die im Kreislauf 1 vorhandene Flüssigkeit durch die Ableitung 71 abgeleitet werden kann, wobei die Abströmung durch den im Behälter 3 vorhandenen Gasdruck und teils durch die Pumpen 7, 77 und 83 und teils durch die Einwirkung der Schwerkraft bewirkt wird. Dabei wird das im Behälter 3 und praktisch das im ganzen Kreislauf 1 vorhandene, flüssige Produkt mindestens im wesentlichen vollständig abgeleitet. Das heisst, die Flüssigkeit wird bis auf allenfalls an Wandungen der den Kreislauf bildenden Bauteile haftende Flüssigkeitsreste abgeleitet, so dass also mindestens 90 %, zweckmässigerweise mindestens 95 % und beispielsweise mindestens 99 % der vorher im Kreislauf zirkulierten Flüssigkeit aus diesem abgeleitet wird. Das Produkt gelangt beim Abströmen aus dem Kreislauf 1 zunächst in den Zwischen-Behälter 75, wo es gespeichert und entgast wird. Sobald der Reaktions-Behälter 3 entleert ist, wird er mit einer frischen, nämlich der zweiten vorgewärmten Edukt-Charge aus dem Zwischen-Behälter 57 gefüllt. Wenn der Zwischen-Behälter 57 leer ist, strömt das aus der

0070797

ersten Edukt-Charge gebildete, im Zwischen-Behälter 75 gespeicherte Roh-Produkt dann durch den Durchgang 55b des Wärmeaustauschers 55 sowie danach durch den Durchgang 49b des Wärmeaustauschers 49 hindurch in den Zwischen-Behälter 79. Gleichzeitig wird aus der Speisequelle 41 flüssiges Edukt für die dritte Charge durch die Durchgänge 49a und 55a der Wärmeaustauscher 49 und 55 hindurchgepumpt.

Dabei durchströmt das Produkt die beiden Wärmeaustauscher 55, 49 im Gegenstrom zum Edukt, wobei Wärme vom Roh-Produkt auf das flüssige Edukt übertragen wird. Das Produkt tritt beispielsweise mit einer Temperatur von ungefähr $200^{\circ}$ C in den Wärmeaustauscher 55 ein, wird beim Durchströmen von diesem auf ungefähr $130^{\circ}$ C abgekühlt, gelangt dann mit dieser Temperatur in den Wärmeaustauscher 49 und wird darin nochmals abgekühlt, so dass es mit einer Temperatur von ungefähr $80^{\circ}$ in den Zwischen-Behälter 79 gelangt. Umgekehrt wird das Edukt beim Durchströmen des ersten Wärmeaustauschers 49 von beispielsweise Raumtemperatur auf ungefähr $110^{\circ}$ C erwärmt, kann im Behälter 51 entgast werden, wird im zweiten Wärmeaustauscher 55 auf beispielsweise $175^{\circ}$ erwärmt und danach im Zwischen-Behälter 57 gespeichert.

Der Kreislauf 1, und insbesondere der Reaktions-Behälter 3, muss zuerst vollständig entleert und eventuell evakuiert werden, bevor die neue Edukt-Charge eingebracht wird. Dies wird dadurch ermöglicht, dass man beim Chargen-Wechsel das flüssige Edukt im Zwischenbehälter 57 und das Produkt oder einen Teil davon im Zwischen-Behälter 75 vorübergehend speichert.

Die folgenden Chargen-Wechsel werden in analoger Weise durchgeführt, wobei jeweils das aus der n-ten Edukt-Charge gebildete Produkt aus dem Kreislauf 1 in den Zwischen-Behälter 75 abgeleitet und danach die n+1-te Edukt-Charge aus dem Zwischen-Behälter 57 in den Reaktions-Behälter 3 eingefüllt wird. Während danach die n+1-te Edukt-Charge im Reaktions-Kreislauf 1 umgewandelt wird, erwärmt das aus der n-ten Edukt-Charge gebildete Produkt in den Wärmeaustauschern 49, 55 die n+2-te Edukt-Charge.

Wenn also die neue Edukt-Charge in den Reaktions-Behälter gelangt, so weist sie bereits die zum Auslösen der Hydrierung erforderliche Minimal-Temperatur oder vorzugsweise die optimale Temperatur auf, so dass sogleich wieder ein Hydrierungsvorgang gestartet und in der bereits beschriebenen Weise durchgeführt werden kann.-

Das aus dem Kreislauf 1 abgeleitete Roh-Produkt wird, wie bereits erwähnt, beim Durchströmen des Wärmeaustauschers 49 auf ungefähr 80° C abgekühlt. Diese Temperatur ist ausreichend tief, um das Roh-Produkt der Filterpresse 85 zuführen zu können. In der eine drehbare Spindel aufweisenden Filterpresse 85 wird das gewünschte Rein-Produkt, d.h. das Amin, vom Katalysator getrennt. Die Filtration läuft dabei in bekannter Weise stufenweise ab, wobei die zuerst anfallende, noch Beimengung enthaltende Vorlauf-Flüssigkeit in den Vorlauf-Behälter 91 gelangt und danach nochmals filtriert wird. Das bei der Filtration gewonnene Rein-Produkt, d.h. das Amin, kann dann über die Leitung 87 entnommen und dem weitern Verwendungszweck zugeführt werden. Der bei der Filtration anfallende Katalysator kann über die Leitung 95 entnommen und wieder verwendet werden.

Diese Filtration des aus einer Nitril-Charge gebildeten Roh-Produktes kann während des Zeitraumes erfolgen, in welchem die neue Nitril-Charge hydriert wird.

Wenn von einer Folge im wesentlichen unterbruchslos nacheinander verarbeiteten Chargen die zweitletzte Edukt-Charge in das Produkt umgewandelt worden ist und aus dem Behälter 3 abgeleitet wird, müsste an sich kein neues Edukt mehr zum Zwischen-Behälter 57 zugeführt werden. Da jedoch das aus der zweitletzten Edukt-Charge und danach auch das aus der letzten Charge gebildete Produkt ebenfalls noch gekühlt werden müssen, kann man zu diesem Zweck eine Kühlflüssigkeit durch die Wärmeaustauscher-Durchgänge 49a, 55a hindurch in den Zwischen-Behälter 57 leiten und danach wieder durch den Reaktions-Behälter 3 hindurch ableiten. Als Kühlflüssigkeit kann beispielsweise das Edukt, das dann im Reaktions-Behälter nicht mehr zur Reaktion gebracht wird, oder eine andere gleichzeitig als Spülmittel dienende Flüssigkeit verwendet werden. Eine Abkühlung dieser Kühlflüssigkeit dürfte im allgemeinen nicht erforderlich sein, kann aber nötigenfalls beispielsweise mittels des Wärmeübertragungsorganes 11 erfolgen.

Im folgenden sollen anhand beispielsweiser Zahlenangaben einige charakteristische Grössen des erfindungsgemässen Verfahrens erörtert und mit den entsprechenden Grössen eines nicht erfindungsgemässen Verfahrens verglichen werden, das mit einem vorbekannten, nicht-erfindungsgemässen Schleifenreaktor durchgeführt werden kann, wie er beispielsweise in der bereits zitierten Publikation "Loop Reactor Technology Improves Catalytic Hydrogenations" von R.J. Malone beschrieben ist.

Als Beispiel wird der Fall betrachtet, dass pro Tag eine Produktmenge von 100 Tonnen hergestellt wird. In der nachfolgenden Tabelle sind nun einige Grössen für die vorbekannte Einrichtung bzw. das erfindungsgemässe Verfahren angegeben. Dabei sind die Dampf- bzw. Kühlwassermengen angegeben, die bei ununterbrochenem Chargen-Betrieb für eine Tagesproduktion erforderlich sind, wobei die Sonderverhältnisse bei den zwei ersten und den zwei letzten Chargen nicht berücksichtigt werden. Im weitern gilt der angegebene Kühlwasserverbrauch unter der Annahme, dass entweder im Kühler des Wärmeregelorgans 37 oder direkt im Wärmeübertragungsorgan 11 Kühlwasser von ungefähr Raumtemperatur auf Siedetemperatur aufgewärmt und verdampft wird.

Tabelle

|  | Stand der Technik | Erfindung |
|---|---|---|
| Volumen des Reaktions-Behälters | $13,5 \text{ m}^3$ | $9 \text{ m}^3$ |
| Dampfverbrauch zum Aufheizen des flüssigen Edukts auf Reaktionstemperatur | 17000 kg | 0 |
| Kühlwasserverbrauch zum Kühlen der durch den Primär-Kreislauf zirkulierenden Flüssigkeit | $300 \text{ m}^3$ | $30\text{-}50 \text{ m}^3$ |

Wie aus der Tabelle ersehen werden kann, hat also die erfindungsgemässe Einrichtung gegenüber einer vorbekannten, für den chargenweisen Betrieb vorgesehene Einrichtung den Vorteil, dass der Reaktions-Behälter 3 kleiner sein kann. Dementsprechend können auch andere Bauteile der erfindungsgemässen Einrichtung kleiner bemessen werden. Ferner wird beim erfindungsgemässen Verfahren, wenn man von der zum Aufheizen der beiden ersten Edukt-Chargen erforderlichen Energie absieht, keine Energie von externen Quellen benötigt, um das flüssige Edukt auf Reaktionstemperatur aufzuheizen. Während beim vorbekannten Verfahren mittels Kühlwasser während des Reaktionsablaufes die anfallende Reaktionswärme abgeführt und das Roh-Produkt danach ebenfalls mittels Kühlwasser noch auf Filtrationstemperatur abgekühlt werden muss, erfolgt das Abkühlen des Roh-Produktes auf Filtrationstemperatur beim erfindungsgemässen Verfahren, mindestens wenn man von den zwei letzten Chargen einer Serie absieht, ohne dass hierfür Kühlwasser benötigt wird. Dementsprechend ist der Kühlwasserverbrauch beim erfindungsgemässen Verfahren wesentlich kleiner, wobei die während der Reaktionsphase freigesetzte Wärme zudem noch in externen Verbrauchern verwendet werden kann.

Abgesehen von der möglichen Verkleinerung verschiedener Bauteile und der Verbesserung der Energiebilanz besteht jedoch der Hauptvorteil des erfindungsgemässen Verfahrens darin, dass die für die Verarbeitung einer Charge erforderliche Gesamtzeit wesentlich verkürzt wird, was dann eben erlaubt mit einem kleineren Reaktions-Behälter auszukommen. Beim erfindungsgemässen Verfahren werden pro Charge 10 Minuten für das Einfüllen des flüssigen Eduktes in den Behälter 3, 74 Minuten für das Durchführen der Reaktion, 10 Minuten für die Entleerung und allfällige Evakuation und eventuell noch im Maximum 15 Minuten für eine allfällige Spülung benötigt. Dies ergibt eine Gesamtzeit von 94 bis 109 Minuten. Beim vorbekannten Verfahren beträgt der Zeitaufwand pro Charge demgegenüber für die in der Einleitung erwähnte Cloranisidin-Herstellung, die was den eigentlichen Reaktionsablauf anbelangt, mit der Amin-Herstellung vergleichbar ist und wegen der kleineren, nur 90° C betragenden Reaktionstemperatur sogar nur eine geringere Edukt-Erwärmung und Produkt-Abkühlung erfordert, 164 Minuten.

Da es beim erfindungsgemässen Verfahren im Gegensatz zum vorbekannten Verfahren (gemäss R.J. Malone) nicht notwendig ist, das Wärmeübertragungsorgan 11 zu benutzen, um das flüssige Edukt auf Reaktionstemperatur aufzuheizen, kann der Kühlvorgang nach dem Einsetzen der chemischen Reaktion im Bedarfsfall sehr schnell eingeleitet werden. Dies gibt auch bei Reaktionen, bei denen beim Beginn der Reaktionsphase schlagartig viel Energie freigesetzt wird, eine hohe Sicherheit gegen das "Durchgehen" des Reaktionsablaufs, d.h. gegen eine zu starke Erhöhung der Temperatur der im Kreislauf 1 zirkulierenden Flüssigkeit.

Nun sollen noch einige Bemerkungen zur Ausbildung der Wärmeaustauscher 49, 55 gemacht werden. Erstens sei festgestellt, dass natürlich jeder der erwähnten Durchgänge 49a, 49b, 55a, 55b aus einer Anzahl parallel verlaufender Teil-Durchgänge gebildet sein kann. Da ferner damit zu rechnen ist, dass in den Durchgängen 55b, 49b Katalysator aus dem Roh-Produkt ausfällt und sich festsetzt, sind die Wärmeaustauscher 49, 55 vorteilhafterweise derart ausgebildet, dass sie für die Reinigung zerlegt werden können. Die Wärmeaustauscher 49, 55 können beispielsweise ein zerlegbares Gehäuse aufweisen, in dem eine Anzahl die Durchgänge begrenzender Platten lösbar gehalten sind. Es sei in diesem Zusammenhang noch bemerkt, dass die Gefahr der Bildung eines Katalysator-Niederschlages im Wärmeübertragungsorgan 11 wesentlich kleiner ist als bei den Wärmeaustauscher 49, 55 weil die Strömungsgeschwindigkeit im Wärmeübertragungsorgan 11 grösser ist als in den Wärmeaustauschern 49 und 55.

Die erfindungsgemässe Einrichtung kann natürlich nicht nur für die beschriebene Hydrierung eines Nitrils, sondern auch für eine Vielzahl anderer chemischer Reaktionen verwendet werden, bei denen ein flüssiges, organisches Edukt und ein gasförmiges Edukt zur Reaktion gebracht werden. Hierbei wären eine Vielzahl von Hydrierungen sowie Äthoxylierungen, Nitrierungen mit $NO_2$ und Carbonylierungen zu erwähnen. Im übrigen sei auch auf die Beispielsangaben in der in der Einleitung zitierten Literatur verwiesen.

Die sich bei der Wärmeübertragung in den Wärmeaustauschern 49 und 55 ergebenden Flüssigkeitstemperaturen können natürlich für verschiedene chemische Reaktionen verschieden sein, bzw. festgelegt werden. Für einen grossen Teil der vorgesehenen Anwendungen dürfte es jedoch zweckmässig sein, wenn das von der Speisequelle 41 her zugeführte, flüssige Edukt im ersten Wärmeaustauscher auf eine mindestens 80° C und höchstens 130° C betragende Temperatur aufgeheizt wird. Im zweiten Wärmeaustauscher soll das zugeführte Edukt danach mindestens auf die zum Auslösen der vorgesehenen Reaktion vorteilhafte Temperatur erhitzt werden. Im allgemeinen dürfte es zweckmässig sein, das zugeführte Edukt im zweiten Wärmeaustauscher auf mindestens 150° C, vorzugweise mindestens 170°, und höchstens 200° C zu erhitzen. Nach dem Anlaufen der Reaktion, kann diese dann bei einer im Bereich von etwa 160 bis 230° C liegenden, konstant gehaltenen Temperatur weitergeführt werden.

Das beim Chargenwechsel aus dem Kreislauf abgeleitete Produkt soll, allenfalls mit Ausnahme der beiden letzten Chargen einer Chargen-Serie, im wesentlichen ausschliesslich durch den Wärmeaustausch mit dem flüssigen Edukt von der Temperatur, die das Produkt am Ende der Reaktionsphase aufweist, auf eine Temperatur abgekühlt werden, die die Filtration in der Filterpresse erlaubt. Das Produkt wird daher nach dem Wärmeaustausch auf eine Temperatur abgekühlt, die höchstens 110$^{\circ}$ C, und vorzugsweise höchstens 90$^{\circ}$ C beträgt.

Die vorzugsweise mindestens zum Teil gleichzeitig mit dem Ablauf einer Reaktion im Kreislauf 1 erfolgende Hindurchleitung oder -förderung des flüssigen Edukts und des flüssigen Produkts durch die beiden Wärmeaustauscher 49, 55 soll derart festgelegt und/oder gesteuert werden, dass das Edukt und das Produkt ungefähr die gleiche Zeitdauer zum Passieren der beiden Wärmeaustauscher benötigen. Dadurch kann erreicht werden, dass mindestens 90 %, vorzugsweise 95 % und beispielsweise 100 % einer Produkt-Charge mindestens einen der beiden Wärmeaustauscher 49, 55 gleichzeitig mit dem Edukt passiert und Wärme an dieses abgeben kann. Umgekehrt kann auch erreicht werden, dass mindestens 90 %, vorzugsweise mindestens 95 % und beispielsweise 100 % einer Edukt-Charge mindestens in einem der beiden Wärmeaustauscher Wärme vom Produkt übernehmen kann.

Die Einrichtung ist vorzugsweise mit einer Steuervorrichtung versehen, die die beschriebenen Arbeitsabläufe automatisch steuert und insbesondere die Ventile 45, 59, 73, 76, 81 öffnet und schliesst sowie eventuell regelt und die Pumpen 7, 47, 53, 77, 83, 107 ein- und ausschaltet und eventuell regelt.

Die Einrichtung ist vorwiegend für die chargenweise Durchführung exotherm ablaufender Reaktionen vorgesehen. Es ist jedoch an sich auch möglich endotherm ablaufende Reaktionen durchzuführen, wobei dann der im Kreislauf 1 zirkulierenden Flüssigkeit während der Reaktionsphase mittels des Wärmeübertragungsorgans 11 Wärme zugeführt werden muss.

Wie vorgängig beschrieben, können die erste und die zweite Edukt-Charge beim Durchströmen der beiden Wärmeaustauscher 49, 55 mit einem im Gegenstrom durch diese hindurchgeleiteten Spül- und Heizmittel erwärmt werden. Man könnte die beiden ersten Edukt-Chargen jedoch auch kalt in den Behälter 3 einleiten und sie jeweils während und nach dem Einleiten im zunächst noch evakuierten, wasserstofffreien Kreislauf 1 zirkulieren und sie dabei mit dem Wärmeübertragungsorgan 11 erwärmen. Wenn die Reaktionstemperatur erreicht ist, kann Wasserstoff eingeleitet und dadurch die Reaktion ausgelöst werden, wobei dann das Wärmeübertragungsorgan 11 zum Kühlen benutzt wird. Da das Wärmeübertragungsorgan 11 bei dieser Variante nur gerade bei den zwei ersten Chargen zuerst zum Aufheizen benutzt wird und danach ausschliesslich zum Kühlen dient, kann auch bei dieser Variante ein "Durchgehen" des Reaktors noch sicher verhindert werden. Das Dreiwegventil 76 könnte für diese Betriebsweise eventuell durch ein abzweigungsfreies Sperr- oder Regelventil ersetzt werden und die Leitung 105 könnte eventuell weggelassen werden.

Des weitern könnte nötigenfalls zusätzlich zum Ventil 59 in den den Zwischen-Behälter 57 mit dem Reaktions-Behälter 3 verbindenden Zuleitungs-Abschnitt noch eine Pumpe eingeschaltet werden. Falls eine allenfalls dort angeordnete Pumpe im Ruhezustand die Zuleitung dicht absperrt und/oder wen der Zuleitungs-Abschnitt zwischen der Pumpe und dem Behälter 3 einen über die Flüssigkeitsniveaus in den Behältern 3 und 57 nach oben ragenden Bogen, einen sogenannten Schwanenhals bildet, könnte dafür eventuell das Ventil 59 wegfallen.

Je nach der Anordnung und Höhe über Boden der Wärmeaustauscher 49, 55, der Speisequelle 41 und der Behälter 51, 57, 75 kann eventuell auf die eine oder andere der Pumpen 47, 53 und 77 verzichtet werden, wobei die Funktion dieser Pumpe dann durch die Einwirkung der Schwerkraft ersetzt würde. Falls hingegen der Zwischen-Behälter 75 oberhalb des Kreislaufs 1 angeordnet würde, könnte nötigenfalls in den Ableitungs-Abschnitt zwischen dem Kreislauf 1 und dem Zwischen-Behälter 75 noch eine Pumpe eingeschaltet werden, wobei dafür eventuell das Ventil 73 wegfallen könnte. Wenn etwa der Wärmeaustauscher 55 oberhalb des Zwischen-Behälters 75 angeordnet würde, könnte eventuell auf ein Ventil zum Sperren und Freigeben des dem Behälter 75 mit dem Wärmeaustauscher 55 verbindenden Ableitungs-Abschnittes verzichtet werden. Es bestehen also verschiedene Möglichkeiten, den Zwischen-Behältern 57 und 75 in der Strömungsrichtung gesehen Mittel vor und/oder nachzuschalten, um in der Zuleitung für das flüssige Edukt und in der Ableitung für das flüssige Produkt nach Bedarf wahlweise Fluidströmungen zu bewirken und/oder zu verhindern, so dass Edukt- und Produkt-Chargen in der vorgängig beschriebenen Art gewechselt werden können und dabei einen Wärmeaustausch zwischen Produkt und Edukt erwirkt werden kann.

Des weitern bestände die Möglichkeit den Zwischen-Behälter 57 und/oder den Zwischen-Behälter 75 durch zwei oder mehr miteinander verbundene Behälter zu ersetzen.

Ferner könnte man eventuell entweder den Zwischen-Behälter 57 oder den Zwischen-Behälter 75 weglassen. Wenn etwa der Zwischen-Behälter 57 nicht vorhanden wäre, könnte eine im Kreislauf 1 vorhandene Produkt-Charge beim Chargen-Wechsel zuerst in dem Zwischen-Behälter 75 abgeleitet werden. Nach der Entleerung des Kreislaufs 1 könnte die Produkt-Charge aus den Zwischen-Behälter 75 durch die Wärmeaustauscher 49, 55 hindurchgeleitet und gleichzeitig die neue Edukt-Charge durch die Wärmeaustauscher hindurch direkt in den Kreislauf 1 eingeleitet werden.

Im übrigen können natürlich die Zuleitung für das flüssige Edukt und die Produkt-Ableitung bei andern Stellen mit dem Kreislauf des Schleifenreaktors verbunden werden, als es in der Zeichnung dargestellt und vorgängig beschrieben wurde.

Des weitern könnte man auch zwei oder noch mehr je einen Reaktions-Kreislauf mit je einem Reaktions-Behälter aufweisende Schleifenreaktoren zur Bildung einer Kaskade fluidmässig hintereinanderschalten und eine in den ersten Kreislauf mündende Zuleitung zum Zuführen eines flüssigen Edukts und eine aus dem letzten Kreilauf abzweigende Ableitung zum Ableiten des gebildeten Produktes vorsehen. Die Zuleitung und die Ableitung könnten dann über mindestens einen Wärmeaustauscher geführt werden, wobei in die Zuleitung und/oder in die Ableitung ein Zwischen-Behälter und weitere Mittel, wie Ventile und/oder Pumpen eingeschaltet sein können, um bei einem chargenweisen Betrieb einen Wärmeaustausch zwischen dem zugeführten flüssigen Edukt und dem abgeleiteten Produkt zu ermöglichen.

Wenn bei einer Einrichtung mit einer Schleifenreaktor-Kaskade Mittel vorgesehen werden, um zwei hintereinandergeschaltete Schleifenreaktoren wahlweise fluidmässig zu verbinden oder zu trennen, kann man eventuell diese Kreisläufe beim Chargen-Wechsel fluidmässig voneinander trennen und beispielsweise den Reaktions-Behälter des fluidmässig letzten Schleifenreaktors bei den Chargen-Wechseln als in die Ableitung des vorgeschalteten Schleifenreaktors eingeschalteten Zwischen-Behälter für die vorübergehende Produkt-Speicherung verwenden. Stattdessen oder zusätzlich könnte auch der Reaktions-Behälter des fluidmässig ersten Schleifenreaktors bei den Chargen-Wechseln als in die Zuleitung zum nachfolgenden Schleifenreaktor eingeschalteter Zwischen-Behälter für die vorübergehende Edukt-Speicherung benutzt werden. Wenn auf diese Weise gewisse Reaktions-Behälter zusätzlich bei den Chargen-Wechseln vorübergehend als Zwischen-Behälter benutzt werden, kann man die zwischen dem Wärmeaustauscher und dem ersten und letzten Kreislauf in die Zuleitung bzw. Ableitung eingeschalteten, ausschliesslich zur vorübergehenden Speicherung dienenden Zwischen-Behälter eventuell kleiner bemessen oder eventuell sogar mindestens einen dieser Zwischen-Behälter ganz weglassen.

Patentansprüche

1. Einrichtung mit mindestens einem einen Kreislauf (1) aufweisenden Schleifenreaktor, einer in den Kreislauf (1) mündenden Zuleitung (43) zum Zuführen eines flüssigen Edukts, einer vom bzw. von einem Schleifenreaktor-Kreislauf (1) abzweigenden Ableitung (71) zum Ableiten eines durch eine chemische Reaktion im Kreislauf (1) gebildeten Produkts und mindestens einem Wärmeaustauscher (49, 55) mit einem in die Zuleitung (43) sowie einem in die Ableitung (71) eingeschalteten oder einschaltbaren Durchgang (49a, 49b, 55a, 55b), dadurch gekennzeichnet, dass Mittel (7, 45, 47, 53, 57, 59, 73, 75, 76, 77) vorhanden sind, um wahlweise derart eine Fluidströmung durch die Zuleitung (43) und die Ableitung (71) zu verhindern oder zu bewirken, dass das Edukt dem Kreislauf (1) chargenweise zugeführt und das Produkt chargenweise aus dem Kreislauf (1) abgeleitet werden und trotzdem ein Wärmeaustausch im Wärmeaustauscher (49, 55) stattfinden kann.

2. Einrichtung nach Anspruch 1, dadurch gekennzeichnet, dass sowohl in die Zuleitung (43) als auch in die Ableitung (71) zwischen dem Kreislauf (1) und dem Wärmeaustauscher (49,55) ein Zwischen-Behälter (57,75) eingeschaltet ist und dass in die Zuleitung (57) zwischen
dem in sie eingeschalteten Zwischen-Behälter (57) und
dem Kreislauf (1) und/oder in die Ableitung (71) vor
und nach dem in sie eingeschalteten Zwischen-Behälter
(75) Mittel (7,45,47,53,59,73,76,77) zum wahlweisen
Absperren der Leitung (43,71) bzw. Durchlassen und/
oder Durchfördern des flüssigen Eduktes bzw. Produktes
eingeschaltet sind.

3. Einrichtung nach Anspruch 1 oder 2, dadurch gekennzeichnet, dass in die Zuleitung (43) ein vorzugsweise
wärmeisolierter Zwischen-Behälter (57) eingeschaltet
ist und dass zwischen diesem und dem Kreislauf (1) ein
Ventil (59) eingeschaltet ist.

4. Einrichtung nach einem der Ansprüche 1 bis 3, dadurch gekennzeichnet, dass in die Ableitung (71) ein
vorzugsweise wärmeisolierter Zwischen-Behälter (75) eingeschaltet ist und dass zw-ischen diesem und dem Kreislauf (1) ein Ventil (73) eingeschaltet ist.

5. Einrichtung nach einem der Ansprüche 1 bis 4, dadurch gekennzeichnet, dass in die Ableitung (71) ein
Zwischenbehälter (75) eingeschaltet ist und dass zwischen
diesem und dem Wärmeaustauscher (55) ein Ventil (76)
eingeschaltet ist.

6. Einrichtung nach einem der Ansprüche 1 bis 5, dadurch gekennzeichnet, dass ein erster Wärmeaustauscher
(49) und ein zweiter Wärmeaustauscher (55) vorhanden
sind, die je einen in die Zuleitung (43) und je einen in
die Ableitung (71) eingeschalteten bzw. einschaltbaren
Durchgang aufweisen und dass die beiden Wärmeaustauscher (49,55) derart in die Leitungen (43,71) eingeschaltet bzw. einschaltbar sind, dass das Edukt zuerst den ersten und danach den zweiten und das Produkt zuerst den zweiten und danach den ersten Wärmeaustauscher durchströmt, wobei vorzugsweise in dem die beiden Wärmeaustauscher (49,55) miteinander verbindenden Abschnitt der Zuleitung (43) ein Entgasungs-Behälter (51) eingeschaltet ist, der mit einer zum Absaugen von Gas dienenden Saugpumpe (31) verbunden ist.

7. Verfahren, bei dem ein flüssiges Edukt durch mindestens einen Wärmeaustauscher (49,55) einem Kreislauf (1) zugeführt und in diesem einer chemischen Reaktion unterzogen wird und ein im Kreislauf (1) gebildetes, flüssiges Produkt aus dem Kreislauf (1) abgeleitet und durch den Wärmeaustauscher (49,55) hindurchgeleitet wird, so dass in diesem Wärme vom Produkt auf das Edukt übertragen wird, dadurch gekennzeichnet, dass das flüssige Edukt dem Kreislauf (1) chargenweise zugeführt und das flüssige Produkt chargenweise aus dem Kreislauf (1) abgeleitet wird und dass bei mindestens einem Teil der Chargen-Wechsel eine Edukt-Charge und/oder eine Produkt-Charge vorübergehend zwischen dem Kreislauf (1) und dem Wärmeaustauscher (49,55) gespeichert wird.

8. Verfahren nach Anspruch 7, dadurch gekennzeichnet, dass mindestens beim genannten Teil der Chargen-Wechsel eine im Kreislauf (1) vorhandene Produkt-Charge mindestens im wesentlichen vollständig aus dem Kreislauf (1) abgeleitet wird, bevor diesem die neue Edukt-Charge zugeführt wird, wobei zweckmässigerweise mindestens 90%, und vorzugsweise mindestens 95%, einer Produkt-Charge im bzw. in einem Wärmeaustauscher (49,55) Wärme an das Edukt abgibt und/oder zweckmässigerweise mindestens 90% und vorzugsweise mindestens 95% der Edukt-Charge im Wärmeaustauscher (49,55) Wärme vom Produkt übernimmt.

9. Verfahren nach Anspruch 7 oder 8, dadurch gekennzeichnet, dass das Edukt beim genannten Teil der Chargen-Wechsel vor seinem Eintritt in den Kreislauf (1) auf eine zum Auslösen der chemischen Reaktion erforderliche und/oder zweckmässige Temperatur erwärmt wird, wobei das Edukt ausschliesslich durch das Produkt auf die genannte Temperatur erwärmt wird und wobei vorzugsweise das bei der chemischen Reaktion im Kreislauf (1) gebildete Produkt von der sich beim Reaktionsablauf ergebenden Temperatur mindestens beim genannten Teil der Chargen-Wechsel im wesentlichen ausschliesslich durch Wärmeaustausch mit dem für die nächste Charge zugeführten, flüssigen Edukt auf eine für eine nachfolgende Filtration zulässige Temperatur abgekühlt wird.

10. Verfahren nach einem der Ansprüche 7 bis 9, dadurch gekennzeichnet, dass dem Kreislauf (1) noch ein gasförmiges Edukt, beispielsweise Wasserstoff, zugeführt wird.